# EUROPEAN PATENT SPECIFICATION

(11) **EP 3 558 149 B1**
(45) Date of publication and mention of the grant of the patent: **24.02.2021**
(21) Application number: 17818181.4
(22) Date of filing: 22.12.2017
(51) Int. Cl.: A61B 18/20, A61B 18/00

(54) **LASER DEVICE FOR TISSUE CHARACTERIZATION**
LASERVORRICHTUNG ZUR GEWEBECHARAKTERISIERUNG
DISPOSITIF LASER POUR LA CARACTÉRISATION DE TISSUS

(30) Priority: 22.12.2016 EP 16206428
(43) Date of publication of application: 30.10.2019
(73) Proprietor: Advanced Osteotomy Tools - AOT AG, 4051 Basel (CH)
(72) Inventor: BRUNO, Alfredo E., 4105 Biel-Benken (CH); BÖRNSEN, Klaus Olaf, 79211 Denzlingen (DE)
(74) Representative: Latscha Schöllhorn Partner AG
(86) International application number: PCT/EP2017/084334
(87) International publication number: WO 2018/115415

(56) References cited:
- WO-A1-01/35881
- US-A1- 2006 084 957
- US-A1- 2009 281 536
- US-A1- 2011 100 967
- US-A1- 2016 317 228

## Description

### Technical Field

The present invention relates to a laser device according to the preamble of independent claim 1 for carrying out a tissue characterizing method.

Such laser devices comprising an ablation laser source adapted to provide an ablating laser beam for ablating a target tissue can be used in many applications such as in medical or surgical applications where a human or animal natural tissue is to cut or to ablate otherwise.

### Background Art

For cutting and drilling human or animal hard or soft tissue such as bones, cartilages or the like various different tools such as saws, chisels or drills are commonly used since a long time. In recent years, also cutting and drilling with laser beam is becoming more and more popular. For example, in some computer assisted surgery it is known to use laser beams as cutting instruments. More particularly, e.g., in WO 2011/035792 A1 a computer assisted and robot guided laser osteotomic medical device is described which allows for precise and gentle drilling and cutting of bone and other human or animal hard and also soft tissue using a mechanism of action that prevents the local heat to reach the cut surfaces rendering faster and stronger healings.

Further, in order to acquire information about the health of human or animal tissues it is standard to use biopsies. Biopsies are tests normally performed by a surgeon, an interventional radiologist, or an interventional cardiologist to determine the presence or extent of a disease. Biopsies usually involve extraction of sample cells or small amounts of tissues for its subsequent examination typically under a microscope by a pathologist using specific reagents. The tissue can also be analyzed or characterized by chemical methods. In the last years increasing automation in medical and research applications optical biopsies became increasingly widespread to reduce the risk or, e.g., recurrence in the case of tumours.

For example, tumour recognition and precise tumour margin detection represents a central challenge during a surgical intervention such as a tumour removal in bone. The surgeon needs to know if the tissue being cut surrounding the tumour is healthy or if it has also cancerous cells. Typically, for this task analysis by biopsy is too slow and the surgeon opt to cut additional tissue in order to enhance certainty that the carcinogen tissue is removed. Indeed, and despite the recent technological advances, biopsies remain time consuming and rather cumbersome procedures. Furthermore, standard biopsies are conducted post-operatively; depending on the results of the biopsy it might be necessary to proceed to a subsequent surgical intervention. In other words, such processes do not allow for reacting during the intervention depending on the outcome of the biopsy as desired by surgeons and patients.

Therefore, there is a need for a device or method allowing the characterization of the tissue as fast and as accurate as possible and particularly within the time of a surgical intervention.

### Disclosure of the Invention

According to the invention this need is settled by a laser device as it is defined by the features of independent claim 1. Preferred embodiments are subject of the dependent claims.

In particular, the invention deals with a laser device having an ablation laser source adapted to provide an ablating laser beam for ablating a target tissue. The laser device comprises a plume analyzing arrangement adapted to identify and/or quantify at least one substance in the debris of the plume generated by the laser beam ablating the target tissue, wherein the substance is a biomarker of the ablated target tissue.

The term "laser device" generally relates to a device which is arranged to generate a laser beam or which emits light through a process of optical amplification based on the stimulated emission of electromagnetic radiation. Laser is an acronym for "light amplification by stimulated emission of radiation". A laser may differ from other sources of light in that it emits light coherently. Such spatial coherence can allow a laser to be focused to a tight spot that bring comparably high power in a variable manner to a surface or the target tissue, which makes applications such as cutting or lithography possible.

The target tissue can particularly be a human or animal natural hard or soft tissue. In particular, the laser device can be open to all kind of tissues, i.e. it can be capable of discriminating various or any tissue in a human or animal being. Thereby, the term "hard tissue" can relate to nail tissue, cartilage and particularly to bone tissue. Thus, the laser device can particularly be designed for or be capable of cutting bones. For being suitable to ablate hard tissue and particularly bone tissue the ablating laser beam can have a wavelength appropriate to evaporate water, i.e. about 2'940 nanometer (nm). It can particularly be a pulsed laser beam. Thereby, the term "laser pulse" can relate to a comparably short-time laser beam preferably of a given wavelength having a specific temporal width, shape and power.

The term "plume" as used herein can relate to a product of a combustion or carbonization process and can comprise odorous molecules, smoke, aerosols and the like referred to as debris. More specifically, in the context of laser ablation, plume can summarize or comprise any substance ejected by the laser beam when hitting the target tissue as debris. Consequently, in connection with plume, the term "debris" can relate to any molecules resulting from the ablation of the target tissue such as volatile small solid fractions of the target tissue, smoke, aerosols, odorous molecules and the like.

The term "substance" as used herein can relate to a single substance, a mixture of plural substances or a pattern of a given number of masses or molecules, any spectroscopic pattern or the like.

The principle of the laser device is that the ablating laser beam provided by the ablating laser source creates a plume with debris. This plume usually is more or less counter-propagating approximately along the direction of the ablating laser beam after every laser pulse of the ablating laser beam has stroke the tissue. Such debris comprises molecules, atoms, fragments of cells as well as ions and electrons in the form of debris. The composition of the debris is indicative of the tissue being ablated. Thus, it can be a characteristic, or a "signature", of the ablated tissue.

By identifying and quantifying the relative concentrations of the debris, or ratios among the intensity of biomarkers or cell fragments associated with healthy and, e.g., cancerous tissue in the plume, it is possible to rapidly decide to enlarge the volume to extract a tumor if it was observed that it is affected with cancerous cells or, to continue the planned operation if the ablated tissue does not show information associated with cancerous cells. Particularly, by analyzing the debris of the plume the identification can be comparably quick and more accurate compared to any other method in which the surface of the target tissue itself is analyzed. This allows for a precise real-time analysis of the target tissue which can, e.g., be integrated in a surgical process.

The laser device according to the invention allows accurately analysing the tissue during a medical and particularly surgical intervention in a comparably fast and accurate manner and, advantageously, within the time of the surgical intervention or in runtime. The proposed device, when being used for in-vivo cancer diagnosis, may eliminate the need of post-operative time-consuming biopsy by an optical biopsy and, thus, to possibly avoid a second intervention.

In combination with a robotic guided system as mentioned above measured in accordance with the invention data of any substance identifying and/or quantifying device such as a spectroscopic device can be collected together with its specific x-, y- and z-position for generating maps of tissue characterization. As described in more detail below, also in combination with optical images overlays augmented reality images or visualizations can be generated.

In comparison with known imaging mass spectrometry (IMS) where in general a fixed positioned detection system is provided and a probe is moved, the laser with its detector or substance identification system can be moving in Y- , Z- and Z-directions. Like this, 3D surfaces can be scanned as well as the interior of laser drilled holes can be analyzed.

In combination with MRI, PET and MRT data, the laser device according to the invention can add three-dimensional surface data of specific areas and can visualize it in all kind of overlay views or augmented images. For example, during a surgery, complete pictures or situations can be visualized and controlled, e.g., to support the surgeon in his dissection. The device according to the invention also allows for taking biopsies with highly precise positions and, therefore, also accurate tumor imaging or mapping becomes feasible. The analytical data together with exact localization can also be used for further calculations, interpolation between data points and for further algorithms, e.g. by neural networking, to develop better visualization skills and for automated strategies for tumor extraction.

The detection or plume analyzation can be combined with statistical analyzing methods. Similar as so-called supervised learning methods the device according to the invention has the potential to do statistical based prediction. At first, before mapping, the system can be trained within the patient supported by a database. On the screen, the different tissues over a given analyzed area can be displayed with its specific statistical accuracy.

The plume analyzing unit can completely or partially be integrated such that the laser device can be a single physical unit. It can also be integrated into another system such as a robotic guided system such that the laser device consists of plural physical units. Further, it can also be embodied as a separate unit. Combinations of these implementations of the plume analyzing unit are also possible.

Preferably, the plume analyzing arrangement comprises a laser spectroscope. Such a laser spectroscope allows for precisely identifying and quantifying substances in the debris of the plume. Such spectroscope also allows for probing the originated plume in real-time with a specific laser beam. Thus, it allows for a comparably quick analysis such that the substances can be identified more or less in real-time time or, at least, within the time of the intervention. The term "real-time" in this connection can relate to an operation of the laser device in which the pulsed ablating laser beam is provided without any restrictions and the plume evaluation is performed during operation. An essential delay and particularly a break in the operation of the laser device is prevented.

The laser device having a laser spectroscope can also be used to determine or analyze the tissue remaining in the surface of the just ablated region instead of the ejected debris in the plume. In specific embodiments of such a laser device, it would even be possible that the plume analyzing arrangement is only capable of analyzing the remaining tissue and that substances in the debris of the plume generated by the laser beam ablating the target tissue are not or not properly identified.

Preferably, the laser device comprises a beam mixing structure, wherein the plume analyzing arrangement has an analysis laser source adapted to provide an analyzing laser beam, and the beam mixing structure is adapted to redirect the ablating laser beam of the ablation laser source and/or the analyzing laser beam of the analysis laser source such that an optical axis of the ablating laser beam is parallel to an optical axis of the analyzing laser beam.

The term "parallel" in connection with the optical axis of the ablating and the analyzing laser beams can relate to a geometrical parallel arrangement, to an orientation essentially parallel, i.e. including minor deviations in alignment, and particularly to an essentially or precisely coaxial orientation of the laser beams.

The beam mixing structure can be an optomechanical structure. Such an optomechanical structure can comprise beam-shaping optics to collimate each individual laser beam, deflection mirrors in the different beam path to allow proper parallel alignment of the laser beams and/or dichroic mirrors. In some embodiments, it can be favorable to have the analyzing laser beam in transmission for the beam mixing structure. To protect optical elements from impurity the beam mixing structure can comprise an out-coupling window after the scanner mirror.

When the beams are mixed coaxially or parallel and considering that in most cases the beams are made up of pulses, the different pulsed beams usually are not propagating in the same space at the same time. In this sense the concept of coaxial or parallel can refer to two pulsed beams propagating through the same space but in slightly different time-periods. Or, one laser can be continuous whereas the other is pulsed such that no such different time-periods occur.

For focusing the composite laser beam the laser ablation device can be equipped with a beam-focusing element which can be comprised by the scanner mirror. The beam-focusing element can be a lens system, reflective optics or a combination of both.

Preferably, the laser device has a movable scanner mirror wherein the scanner mirror is arranged to direct the analyzing laser beam and the ablating laser beam when having parallel optical axes. The term "movable" in connection with the scanner mirror relates to being displaceable and/or particularly re-orientable. For example, the scanner mirror can be rotated, relocated, tilted, bent or the like in order to be movable. Such movable scanner mirror allows for precisely directing and/or focusing the laser beams or composite laser beam.

The term "positioned after the beam mixing structure" in connection with the movable scanner mirror particularly relates to an arrangement or positioning with respect to a direction of laser beam propagation. In particular, the scanner mirror is positioned such that the laser beams first propagate thorough the beam mixing structure and thereafter reach the scanner mirror. In this way, it can be achieved that the mixed laser beams or a composite laser beam is redirected by the scanner mirror.

The scanner mirror as reflective optics can be adapted to focus the cutting laser beam and the analyzing laser beam. Thereby, the scanner mirror can be a concave or parabolic mirror mounted on a movable scanning unit which can simplify alignment and controlling. Such a reflective optics design has further the advantage of smaller losses and no chromatic aberrations when using different wavelengths. Like this, a particular efficient operation of the laser device is possible.

Preferably, the laser spectroscope comprises a laser induced fluorescence (LIF) spectroscope, a coherent anti-Stokes Raman scattering spectroscope (CARS), a laser photo-acoustic spectroscope (LPAS), a laser induced breakdown spectroscope (LIBS), an atomic emission spectroscope (AES), a AES/LIBS, a resonance-enhanced multi-photon ionization (REMPI) spectroscope, a mass spectroscope (MS), a system where molecules are separated by their collision cross section such as a ion mobility spectroscope (IMS), or an elastic scattering (ES) spectroscope. The choice of a particular laser spectroscope can depend on the specific problem at hand. Also, in some applications it might be advantageous to combine plural of these laser spectroscopes in one single laser device. For example, a combination of optical coherence tomography (OCT), LIBS and mass spectrometry (MS) can be particularly beneficial.

In the following, possible embodiments of the mentioned spectroscopes or the techniques underlying those spectroscopes are described:

Laser induced fluorescence (LIF): LIF is an established method to identify and quantify the concentration of molecules or atoms in the gas phase and, thus, more appropriate for the purpose at hand. It is already successfully applied for quantitative measurement of concentrations in fields like combustion, plasma, spray and flow phenomena. Two different kinds of spectra can be recorded with such an embodiment of the laser device, disperse spectra and excitation spectra.

The disperse spectra are obtained with a fixed excitation wavelength while the fluorescence emission spectrum is analyzed with, e.g., a monochromator. Or, the light of interest is selected by means of optical filter. The excitation with a fixed wavelength could also be achieved by multi-photon excitation such as two-photon excitation requiring usually very short pulses of light. In both cases the coupling of the light into a single coaxial beam is identical.

Excitation spectra, on the other hand, are obtained by collecting fluorescent light at a fixed emission wavelength or range of wavelengths while the wavelength of the excitation laser is changing using a tunable laser for excitation. This mode of operation could be used to select the interesting spectral regions for a given biomarker and, once the optimal spectral parameters have been found the device is operated in the disperse excitation mode.

Coherent anti-Stokes Raman spectroscopy (CARS): CARS basically identifies molecular resonances such as vibrational information in molecules. It is an enhanced form of Raman spectroscopy also sensitive to the vibrational signatures of molecules, and can be suitable for the purpose of, e.g., identifying and quantifying cancer cells. CARS can be used for species selective microscopy and combustion diagnostics. More recently, CARS has been utilized as a method for non-invasive imaging of lipids in biological samples, both in vivo and in vitro.

The general principle of CARS is that multiple photons are used to excite the molecular vibrations, and to produce a coherent signal. CARS is a third-order nonlinear optical process involving three laser beams: a pump beam of frequency ωₚ, a Stokes beam of frequency ω_{S} and a probe beam at frequency ωₚᵣ. These beams interact with the sample and generate a coherent optical signal at the anti-Stokes frequency (ωₚᵣ+ωₚ-ω_{S}). The latter is resonantly enhanced when the frequency difference between the pump and the Stokes beams (ωₚ-ω_{S}) coincides with the frequency of a Raman vibrational resonance.

With CARS not only the components of the debris in the plume can be anaylzed but also the targeted sample or target tissue between to subsequent pulses of the ablating laser beam and, the use of a scanner can allow reconstructing an image of the cellular structure within seconds as the surgical intervention takes place, i.e. real-time.

Laser photo-acoustic spectroscopy (LPAS): LPAS is based on the detection of an acoustic signal generated by a laser when it ablates a tissue. The absorbed energy from the excitation laser light, which in the present laser ablating device can induce local heating followed by an explosion due to the thermal expansion, can generate a pressure wave propagating in the nearby tissue and a sound wave propagating on the open space. In either case, the pressure or the acoustic wave frequencies, amplitude and decay characteristics can be indicative of the tissue being ablated. When the ablating laser beam strikes the tissue evaporating it, the acoustic signal from the explosion can be comparably strong and characteristic for the tissue being ablated. In particular, if the tissue in question is bone the acoustic signal typically has a different signal than when the tissue being ablated is soft tissue or other hard tissue because the acoustic impedance is different in different tissues.

LPAS could also be used to analyze the content of the plume using a separate laser lasing at a wavelength that could be absorbed by a biomarker in the debris of the plume. The set up to bring the LPAS excitation light could be either by means of an optical fiber which is collimated and coaxially mixed with the ablating laser. The detection of the LPAS signal when sampling the plume can be detected by means of a microphone and there can be a few modes to detect and analyze the signal.

Laser-induced breakdown spectroscopy (LIBS): LIBS is a type of atomic emission spectroscopy which uses a highly power laser pulse as the excitation source. The laser typically is focused to form plasma, which atomizes and excites samples. The formation of the plasma only begins when the focused laser achieves a certain threshold for optical breakdown, which generally depends on the environment and the target material. In principle, LIBS can analyze any matter regardless of its physical state, be it solid, liquid or gas because all elements emit light of characteristic frequencies when excited to sufficiently high temperatures. When the components of a material to be analyzed are known, LIBS may be used to evaluate the relative abundance of each constituent element, or to monitor the presence of impurities. In practice, detection limits are a function of a) the plasma excitation temperature, b) the light collection window, and c) the line strength of the viewed transition.

Generally, LIBS is an atomic emission spectroscopic technique that uses a high energy laser pulse as excitation source. It operates by focusing the laser onto a comparably small area at the surface of the specimen; when the laser is discharged it ablates a very small amount of material, in the range of nanograms to picograms, which generates a plasma with temperatures in excess of a specific temperature such as 700 K or 100'000 K depending on type of atoms or molecules. During data collection, typically after local thermodynamic equilibrium is established, plasma temperatures range from 1'000 K to 20'000 K. At the high temperatures during the early plasma, the ablated material dissociates (breaks down) into excited ionic and atomic species. During this time, the plasma emits a continuum of radiation which does not contain any useful information about the species present, but within a very small timeframe the plasma expands at supersonic velocities and cools. At this point the characteristic atomic emission lines of the elements can be observed. The delay between the emission of continuum radiation and characteristic radiation is in the order of 10 µs, this is why it is necessary to temporally gate the detector.

LIBS is technically very similar to a number of other laser-based analytical techniques, sharing much of the same hardware. These techniques are the vibrational spectroscopic technique of Raman spectroscopy, and laser-induced fluorescence (LIF). In fact devices are now being manufactured which combine these techniques in a single instrument, allowing characterization of a sample. A typical LIBS system can comprise of a Nd:YAG solid-state laser and a spectrometer with a wide spectral range and a high sensitivity, fast response rate, time gated detector. With a wavelength of 1'064 nm and pulse duration around of 10 ns it can generate a power density exceeding 1 GW·cm⁻² at the focal point. This can be coupled to a computer to process and interpret the acquired data. As such LIBS is one of the most experimentally simple spectroscopic analytical techniques, making it one of the cheapest to purchase and to operate. Because such a small amount of material is consumed during the LIBS process the technique is considered essentially non-destructive or minimally-destructive, and with a total average power of less than one watt at the target there is almost no heating surrounding the ablation site. LIBS is also a very rapid technique giving results within seconds, making it particularly useful for the purpose at hand, i.e. real-time. LIBS is an entirely optical technique, therefore it requires only optical access to the specimen. And being an optical technique it is non-invasive, non-contact and can be easily integrated into a laser based surgical device.

Resonance-enhanced multi photon ionization (REMPI) spectroscopy: REMPI is usually generated by a focused frequency tunable laser beam to form a small-volume plasma. In REMPI, first m photons are simultaneously absorbed by an atom or molecule in the sample to bring it to an excited state. Other n photons are absorbed afterwards to generate an electron and ion pair. The so-called m+n REMPI is a nonlinear optical process, which can only occur within the focus of the laser beam. A comparably small volume plasma is formed near the laser focal region. If the energy of m photons does not match any state, an off-resonant transition can occur with an energy defect ΔE, however, the electron is very unlikely to remain in that state. For large detuning, it resides there only during the time Δt.

Preferably, the plume analyzing arrangement comprises a mass spectrometer. The mass spectrometer can be capable of analyzing samples at atmospheric pressure. It can be a comparably compact device which advantageously is capable of being conveniently integrated into the laser device. The mass spectrometer can be provided in the laser device in addition to a spectroscope or a combination of spectroscopes.

Generally, mass spectrometric methods or techniques measure mass-to-charge ratio of molecules, fragments of molecules and particles by using electric and magnetic fields to separate them and detected with a suitable detector. When using a mass spectrometric method to analyze the laser generated plume during an, e.g., osteotomic intervention it is thus possible to, e.g., differentiate cancer from normal bone or cancellous, i.e. trabecular or spongious, bone from cortical bone by identifying biomarkers or fragments associated with their respective tissues. There are various suitably types of mass spectrometers based primarily on their principle of operations and required mass resolution.

For the purpose at hand, the identification of the tissue being ablated during an intervention in real-time to avoid, e.g., a biopsy that takes much longer the so-called aspirating ion-mobility spectrometers (AIMS) or also mass spectrometers (MS) can be beneficial. For example, such devices are typically working without or with very small vacuum such that there is no need for comparably bulky vacuum pumps or the like. This allows the devices to be comparably robust and suitable for a clinical environment. Ion-mobility spectrometers separate molecules by their collision cross section (geometric size), but usually have a relatively high sensitivity. Such mass spectrometers can be comparably compact facilitating their integration. They can analyze samples at atmospheric pressure or lower, such as at 100 mbar or even lower, and require very little or even no sample preparation. Such devices are used to separate and identify ionized molecules in the gas phase based on their mobility in a carrier buffer gas. AIMS devices are widely used in airports for security purposes to detect drugs and explosives by aspirating samples at atmospheric pressure very quickly.

The plume analyzing arrangement preferably comprises a debris gathering unit arranged to collect debris of the plume generated by the ablating laser beam when ablating the target tissue wherein the debris gathering unit is connected to the mass spectrometer. Such debris gathering unit can allow for efficiently forwarding the debris to the mass or other spectrometer such that an accurate and fast analysis can be achieved.

Thereby, the debris gathering unit of the plume analyzing arrangement preferably comprises an aspirating mouthpiece. By the aspirating mouthpiece the debris in the plume can be collected and directed to the mass spectrometer, a chromatograph or any other suitable detector. Also, a combination of both where the components of the collected sample is separated by chromatography and further analyzed by mass spectrometry is possible. When using this aspiration or suction approach the outcome can be slightly delayed with relationship to the ablation but certainly within an useful time-scale for the operator of, e.g., less than a few minutes such as some milliseconds or the like.

The debris gathering unit of the plume analyzing arrangement preferably comprises a pump unit adapted to forward the debris to the mass spectrometer. Such a pump unit allows for efficiently forwarding the debris to the mass spectrometer. As an alternative to the pump unit, a vacuum unit may provided in order to forward the debris. Also, pump and vacuum units may be combined. The pump or vacuum units can further be integrated in the plume analyzing arrangements. For example, some plume analyzing arrangement such as, e.g., many mass spectrometers have pump or vacuum units which can be used for forwarding the debris.

Further, the debris gathering unit of the plume analyzing arrangement preferably comprises an electrical field generator to collect the debris of the plume. Such field generator allows for efficiently forwarding the debris on the basis of electrostatic forces.

Preferably, the laser device comprises a processing unit adapted to identify a movement of the target tissue relative to the laser source on the captured image and to correct a position of the laser source in accordance with the identified movement of the target tissue. Such embodiment allows for recognizing movements of the target tissue or patient. Like this it is possible to stop the laser beam when it would not hit the correct defined position on the target tissue. To recognize any movement of the patient, reflector systems may be fixed to the patient or target tissue close to the operation area.

Preferably, the plume analyzing arrangement comprises a processing unit adapted to evaluate measurement data of the at least one substance in the debris of the plume generated by the laser beam ablating the target tissue. Such evaluation can, e.g., comprise a statistical analysis in real-time, which can include all kind of statistical learning and prediction methods. For example, supervised learning techniques and principles (PCA) can be applied. Additionally, statistical comparison with a tissue database can be implemented for identification and risk assessment.

Thereby, the processing unit can be equipped and provided with the necessary structure such as connections to a power supply, a central processing unit (CPU), a memory and the like. Furthermore, it or its structure can be adjusted, adapted or programmed to perform the necessary functions.

The processing unit can be implemented in any manner suitable for achieving its function or purpose. Advantageously, it is an embedded system integrated in the electronics of the laser device. For example, it can be integrated, e.g. as a specific circuit, on the circuit board controlling the laser source. Like this, a particular fast evaluation of the emission light is possible.

The processing unit can comprise several interfaces to internal and external components such as a nozzle, a temperature camera, the laser sources and the like. Advantageously, it has an external user interface to define cutting geometries, set parameters and visualize progress and system status. The laser device may be combined with additional actuating devices to increase working range and tracking devices to follow movement of working range and or actuating device. This may require additional interfaces in the controlling unit to these devices. The processing unit allows for automatically evaluating the information gathered by the plume analyzing arrangement. Thereby, as a result of this evaluation the processing unit can automatically take any measures. For example, the laser beam intensity may be adjusted in relation to the kind of analysis or cutting mode. Or, a surface of the target tissue can be scanned with a comparably low energy laser beam for determining boundaries of a specific type of tissue such as cancerous tissue. Or, the processing unit may calculate a next position, e.g. in x-, y-, z-coordinates, for taking the next tissue analyzing point and the analytical data are visualized on screen.

A further aspect of the present disclosure relates to a tissue characterizing method comprising the steps of: providing an ablating laser beam to a target tissue wherein the ablating laser beam ablates the target tissue such that a plume comprising debris of the target tissue is generated; and identifying and/or quantifying at least one substance in the debris of the plume generated by the laser beam ablating the target tissue.

Such a method and its preferred embodiments allow for efficiently achieving the effects and benefits mentioned above in connection with the laser device according to the invention and its preferred embodiments.

Within the tissue characterizing and/or quantifying method, the at least one substance in the debris of the plume generated by the laser beam ablating the target tissue preferably is identified by a laser spectroscope.

Preferably, the tissue characterizing method comprises the step of providing an analyzing laser beam and redirecting the ablating laser beam and/or the analyzing laser beam such that an optical axis of the ablating laser beam is parallel to an optical axis of the analyzing laser beam.

Thereby, it preferably further comprises the step of directing the analyzing laser beam and the ablating laser beam when having parallel optical axes.

Preferably, the laser spectroscope is a laser induced fluorescence spectroscope, a coherent anti-Stokes Raman scattering spectroscope, a laser photo-acoustic spectroscope, a laser induced breakdown spectroscope, a resonance-enhanced multi-photon ionization spectroscope or an elastic scattering spectroscope.

Preferably, the at least one substance in the debris of the plume generated by the laser beam ablating the target tissue is identified by a mass spectrometer. Thereby, the tissue characterizing method preferably comprises the step of collecting debris of the plume generated by the ablating laser beam laser beam when ablating the target tissue and forwarding the collected debris to the mass spectrometer. Thereby, the collected debris preferably is pumped to the mass spectrometer for forwarding the collected debris to the mass spectrometer.

Preferably, the tissue characterizing method comprises the step of evaluating measurement data of the at least one substance in the debris of the plume generated by the laser beam ablating the target tissue.

Preferably, the tissue characterizing method is not a method for treatment of the human or animal body by surgery or therapy, nor a diagnostic method practiced on the human or animal body. Such a method can be, e.g., a method of analyzing a tissue extracted from the human or animal body. Or, it can be a method for analyzing a cell culture or the like.

Preferably, the laser device comprises a camera adapted to capture an image of the target tissue. The image can be a single image or shot. Or it can be a series of pictures consecutively obtained such that, e.g., a video or movie of the target tissue can be generated. Such a camera can be implemented in various functions of the laser device. For example, it can be used for observing correct application of the device and correct positioning of the target tissue.

Preferably, the plume analyzing arrangement is adapted to three-dimensionally localize the origin of the plume. Particularly, the laser device may be adjusted to precisely determine where the ablation of the target tissue is occurring. For example, this can be defined by adjusting power, direction and origin of the laser beam.

Particularly, the plume analyzing arrangement preferably is adapted to augment the image captured by the camera with information derived from the substance in the debris of the plume. Like this, the information about the substance can be associated to the image of the target tissue. For example, the type of the substance or the substance itself can be three-dimensionally displayed in the image. In particular, such display can be performed more or less runtime such that a practitioner continuously knows what kind of tissue is ablated by the laser beam. This, e.g. allows for precisely knowing when a cancer tissue is ablated and when the end of the cancerous tissue is reached.

Preferably, the ablation laser source is three-dimensionally movable, particularly, relative to the target tissue. More specifically, the ablation laser source can be movable along and/or about an x-axis, a y-axis and a z-axis. For example, the x- and y-axes can be in a plane more or less parallel to a surface of the target tissue and the y-axis can be directed towards the target tissue (depth). Like this, the laser beam can be precisely directed to and positioned at any suitable location of the target tissue. This allows for the target tissue being fixed or stationary and the laser beam moving along the target tissue such that ablation can efficiently be induced at any desired location on the target tissue.

In particular, the ablation laser source can be mounted to a robotic arm or the like such as the arm of a cold ablation robotic laser osteotome (CARLO) as described in WO 2011/035792 A1. Such robotic arm allows for accurate three dimensional movement of the ablation laser source such that natural movements of the target tissue or of a patient, e.g., due to breathing, can be compensated. The laser system of CARLO can stay permanently on any position on the three-dimensional surface of the patient including during surgeries within the body.

When being three dimensionally movable, e.g., by the robotic system or the robotic arm, the laser device can be used to scan or to map any surfaces, i.e. the target tissue, of the patient. When implemented in a CARLO the built-in laser system comprising the ablation laser source can provide precise x-, y- and z-coordinates. The target tissue or the patient can be fixed or stationary and only the robot or ablating laser source moving. The advantage is that in this way a very precise local position of a three-dimensional target tissue, e.g. a patient, can be achieved.

The ablation laser source can be controlled in a way that it can be used for cutting bones and tissues as well as to do probing or scanning. For probing and scanning the laser pulse or beam pumps energy into the surface of the target tissue at comparably low energy. Thereby, the surface molecules such as proteins, lipids small molecules and fragments of them, can be excited in one or the other way. The suitably sensitive plume analyzing arrangement can then analyze these surface molecules and create a map or image of the surface.

Using a CARLO allows for adapting the laser intensity from comparably high energy for cutting bones to comparably low intensity sufficient to analyze spots on the surface by a given laser pulse but not harming the surface. During cutting a bone and also during surface analyzing, the laser pulse can create plume comprising the debris. Depending on the laser intensity, the extent of the plume and debris generation varies resulting in different stamping or characteristics which can be considered by the plume analyzing arrangement in evaluation of the debris. Specifically, these kind of plume and debris can be analyzed with known analytical methodologies like mass spectrometric or ion mobility instruments sucking in parts of the debris or optical spectroscopic instruments analyzing the created light of the plume or debris, like atomic emission spectroscopy, LIBS and other fluorescence effects.

An important advantage of the laser device according to the invention particularly when being embodied in a CARLO is the potential to create two- or three-dimensional images of any given area of the target tissue or the patient body. This can be useful during surgeries to provide enough information about tumor boundaries or any other tissue differences, like nerves, bones, collagen etc., within comparably short time frames. This allows for an online analysis, e.g. in milliseconds to minutes, and may include statistical spectra analyzing methods or comparison with databases for the correct identification of the different kind of tissues.

All these data can be displayed graphically on a computer screen by their correct y-, x- and z-position since the exact position of each collected data point can be derived from the adjustment of the ablation laser source. Together with an optical image, e.g. obtained by a camera, of a given patient surface area, i.e. the target tissue or surgery area, and overlay can be created with one or more data sets collected from combined analytical methods.

Analytical as well as biological tissue differences, particular within a tumor may be very minor. However, it is very important that the analytical device provides enough information to separate tumor from healthy tissue. Also, it may be difficult to identify a given tissue by a single biomarker. Only a set of biomarkers or a given spectroscopically pattern may provide the needed information. It may also be possible, that different analytical methods may be combined to achieve enough information for the statistical separation or identification of the different tissues.

Another aspect of the present disclosure relates to a method of cutting a human or animal natural or artificial hard or soft target tissue (cutting method) comprising the steps of: (a) providing an ablating laser beam for ablating the target tissue; and (b) identifying and/or quantifying at least one substance in the debris of the plume generated by the ablating laser beam when ablating the target tissue. Particularly, the at least one substance can be a biomarker of the ablated target tissue.

The cutting method allows for efficiently achieving the effects and benefits described above in a medical or surgical application. Also it can be embodied with the features described above in order to achieve further effects and benefits.

In particular, in the cutting method the at least one substance preferably is identified by a laser spectroscope, a mass spectrometer or a similar apparatus of a plume analyzing arrangement. Preferably, the cutting method further comprises evaluating measurement data of the at least one substance in the debris of the plume generated by the laser beam ablating the target tissue.

### Brief Description of the Drawings

The laser device according to the invention is described in more detail herein below by way of exemplary embodiments and with reference to the attached drawings, in which:
- Fig. 1: shows a setup of a first embodiment of a laser device according to the invention suitable for performing a tissue characterizing method according to the disclosure;
- Fig. 2: shows a laser spectroscope of the laser device of Fig. 1;
- Fig. 3: shows a beam mixing structure of the laser device of Fig. 1;
- Fig. 4: shows a robotic laser system comprising the laser device of Fig. 1 in operation where the robotic laser system can reach any position on a three dimensional surface of a patient within a correct angle (laser surface and distance); and
- Fig. 5: shows a beam mixing structure of a second embodiment of a laser device according to the invention.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

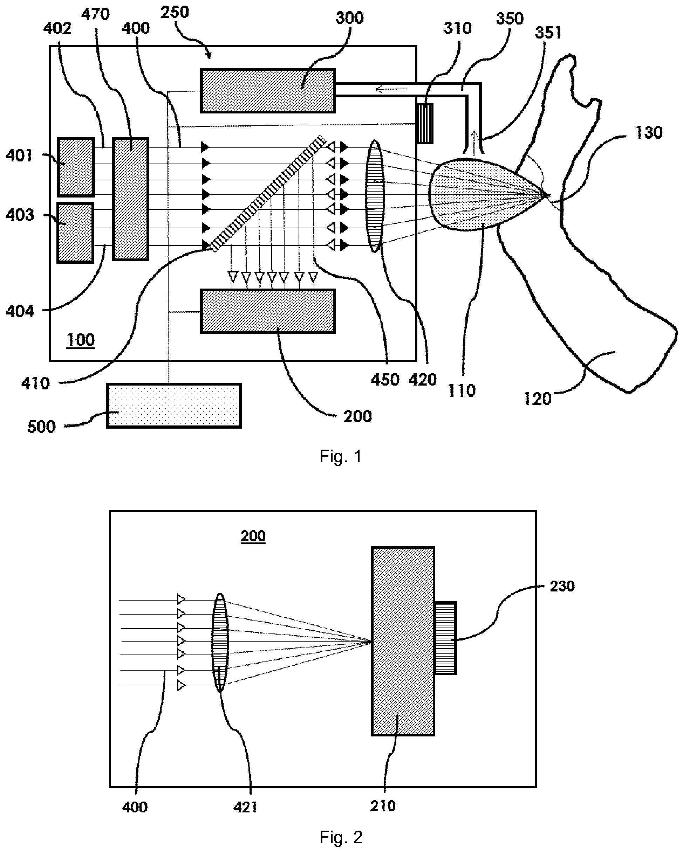

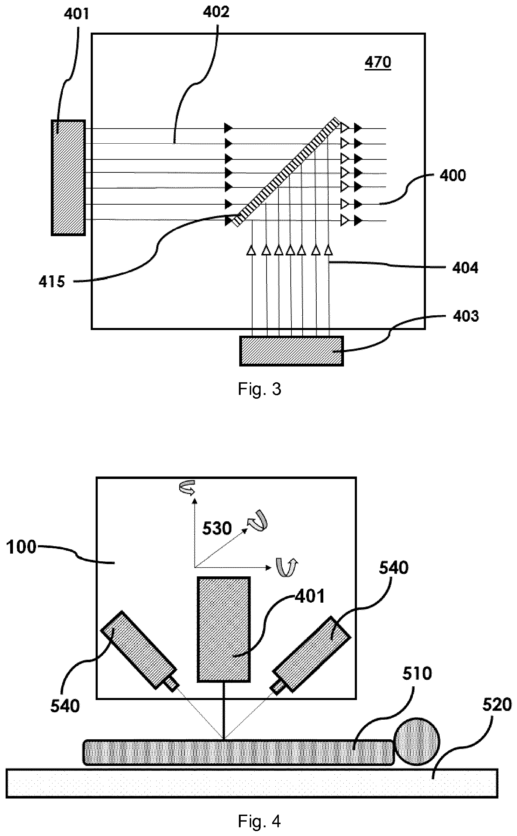

Fig. 1 shows a first embodiment of a laser device 100 according to the invention, e.g. embedded into a cold ablation robotic laser osteotome (CARLO). The laser device 100 comprises an ablation laser source 401 adapted to provide an ablating laser beam 402 for ablating a bone 120 as target tissue. It is further equipped with a beam mixing structure 470 and an analysis laser source 403 arranged to provide an analyzing laser beam 404 at a more or less single wavelength to record the disperse spectra or at a wavelength tuned to record excitation spectra. The beam mixing structure 470 is embodied to mix the ablating laser beam 402 and the analyzing laser beam 404 to a composite laser beam 400 as described in more detail below in connection with Fig. 3.

The laser device 100 further comprises a plume analyzing arrangement 250 with a mass spectrometer 300, a debris gathering unit 350, a microphone 310 and a laser spectroscope 200. The plume analyzing arrangement 250 is embodied to identify and to quantify substances in the debris of a plume 110 generated by the composite laser beam 400 ablating the bone 120 along a predefined osteotomic line 130 after being focused by a beam focusing optics 420.

In use of the laser device 100, the ablating laser beam 402 mixed together with the analyzing laser beam 404 to the composite laser beam 400 passes a dichromatic mirror 410 of the plume analyzing arrangement 250 and is provided to the bone 120 via the beam focusing optics 420. Thereby, the plume 110 comprising debris of the ablated bone tissue is generated. A specific portion of the analyzing laser beam 404 contained in the composite laser beam 400 is reflected by the debris of the plume 110. This reflected light 450 travels more or less in an opposite direction than the composite laser beam 400 away from the bone 120. Thereby, it hits the dichromatic mirror 410 which redirects the reflected light 450 towards the laser spectroscope 200. There, substances of the debris of the plume 110 are identified and/or at least the spectra are recorded.

Simultaneously, the debris of the plume 110 generated by the composite laser beam 400 is collected or sucked by an aspirating mouthpiece 351 of the debris gathering unit 350 and forwarded to the mass spectrometer 300 by means of a pump. There, the debris is analyzed by using acoustic signals sensed by the microphone 310. Like this, substances of the debris are identified.

Further, a processing unit 500 equipped with suitable interfaces is connected to the various components of the laser device 100. The processing unit 500 adjusts the laser sources 401, 403 and evaluates the measurement results of the microphone 310, the mass spectrometer 300 and the laser spectroscope 200.

As shown in Fig. 2 laser spectroscope 200 comprises a light focusing optics 421 which focuses the reflected light 450 redirected by the dichromatic mirror 410. The focused reflected light is provided to a monochromator 210 which is associated to a light detector 230 which can be a photomultiplier, a photodiode, a charged-coupled device or a similar device.

The analyzing laser beam 404 is adapted to induce reflection or emission such as fluorescence or phosphorescence from the debris of the plume 110. Such emission or reflection advantageously propagates coaxially or parallel to composite laser bream 400. Thereby the reflected light 450 or excitation laser beam can be coming from a continuous wave (cw) or from a pulsed laser. If the excitation laser beam is from a cw laser beam the emission could be time-gated to select light right after the ablation laser pulse has reached the bone 120. If the excitation laser beam 450 is from a pulsed laser source it could be time synchronized in such a way that it arrives after a give short time interval to facilitate the discrimination of the emission in the laser spectrometer 200. When interested in the emission of the targeted surface of the osteotomic line 130 or surgical ablating trajectory the emission of the excitation laser beam 450 should be detected using time-gating methods just before the ablating laser reach the target when using a cw laser or, when using a pulsed laser source its pulse should arrive before the pulse of the ablating laser source 401.

Fig. 3 shows the beam mixing structure 470 of the laser device 100 in more detail. It is equipped with a further dichromatic mirror 415 which is transparent for the ablating laser beam 402 and reflective for the analysing laser beam 404. The further dichromatic mirror 415 redirects the analyzing laser beam 404 into the same direction as the ablating laser beam 402. The two laser beams 402, 404 now have parallel or coaxial optical axes and the composite laser beam 400 is generated.

In Fig. 4 the laser device 100 is shown implemented in a robotic laser system such as a CARLO. The laser device 100 and, particularly, its ablation laser source 401 and/or optical elements 410, 420 are mounted to a robotic structure such as a robotic arm. The robotic structure can provide movements in six degrees of freedom in a motion coordinate system 530. In particular, the robotic structure can allow for a movement along and about each of x-, y- and z-axes.

The laser device 100 comprises two cameras 540 adapted to capture an image of the target tissue 120 used for correct positioning and controlling of the position of the leaser beam during patient or target tissue 120 moving such as breathing. To be capable of recognizing smallest movements like breathing two cameras 540 are used. All recognized movements are corrected by a corresponding movement of the laser device 100. The target tissue 120 forms part of a patient 510 which is stationary positioned on a surgical bench 520. The processing unit 500 of the plume analyzing arrangement 250 is adapted to evaluate measurement data obtained of the debris of the plume 110 which is generated by the ablating laser beam 402 ablating the target tissue 120 of the patient 510. Thereby, the plume analyzing arrangement 250 is adapted to three-dimensionally localize the origin of the plume 110 and to augment images captured by the cameras 540 with information derived from debris of the plume 110.

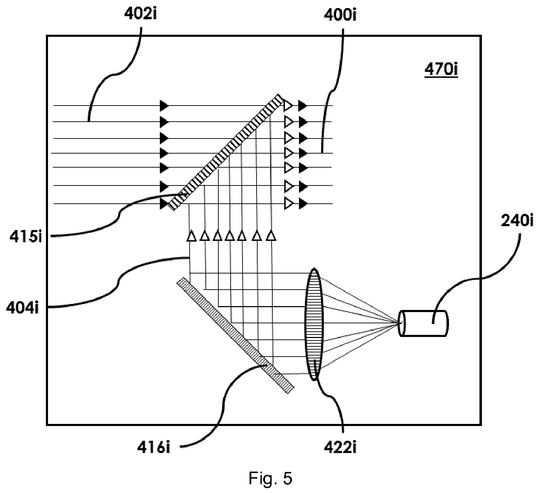

Fig. 5 shows a section of a second embodiment of a laser device according to the invention. This laser device has an essentially identical setup as the laser device 100 described above. In contrast to the first embodiment of the laser device 100 the second laser device is embodied for CARS.

CARS requires two synchronized comparably fast laser pulses at two different wavelengths to generate the Raman response. In the second laser device the two wavelength excitation light 404i from a compact solid state fast laser is coupled to an optical fiber 240i and brought to the second laser device by the same fiber for its easy integration after proper collimation with a collimator 422i and added to the ablating laser beam 402i via a mirror 416i and a further dichromatic mirror 415i to form a composite laser beam 400i. The emission from debris in a plume is captured and analyzed preferably by the same arrangement as described above or, by means of an optical fiber close to the plume at the region of the excitation and directed to a light detector.

When CARS is used to obtain a two-dimensional microscopic CARS emission image of the surface of the ablated tissue the excitation light advantageously is scanned over the region of interest between the pulses of the ablating laser pulses by preferably by means of a scanner or by means of mounting the whole laser ablating device in e.g. a robot or an xyz linear scanner. The frequency of the ablating laser pulses 402i and those of the CARS laser 404i pulses are not necessarily the same. In the shown embodiment the CARS laser frequency is much higher implying that between two subsequent pulses of ablating laser pulses there will be several CARS excitation pulses which are used to improve the signal/noise ratio in the case the CARS laser is probing the particles in the plume or, to make two-dimensional scans if interested in recording the emission of the surface to reconstruct an image.

This description and the accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the scope of this description and the claims. In some instances, well-known circuits, structures and techniques have not been shown in detail in order not to obscure the invention. Thus, it will be understood that changes and modifications may be made by those of ordinary skill within the scope of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. Laser device (100) with an ablation laser source (401) adapted to provide an ablating laser beam (402; 402i) for ablating a target tissue (120) **characterized by** comprising a plume analyzing arrangement (250) adapted to identify and/or quantify at least one substance being a biomarker of the ablated target tissue (120) in debris of a plume (110) generated by the ablating laser beam (402; 402i) ablating the target tissue (120).

2. The laser device (100) of claim 1, wherein the plume analyzing arrangement (250) comprises a laser spectroscope (200).

3. The laser device (100) of claim 2, comprising a beam mixing structure, wherein the plume analyzing arrangement (250) has an analysis laser source (403) adapted to provide an analyzing laser beam (404; 404i), and the beam mixing structure (470; 470i) is adapted to redirect the ablating laser beam (402; 402i) of the ablation laser source (401) and/or the analyzing laser beam (404; 404i) of the analysis laser source (403) such that an optical axis of the ablating laser beam (402; 402i) is parallel to an optical axis of the analyzing laser beam (404; 404i).

4. The laser device (100) of claim 3, comprising a movable scanner mirror positioned after the beam mixing structure (470; 470i), wherein the scanner mirror is arranged to direct the analyzing laser beam (404; 404i) and the ablating laser beam (402; 402i) when having parallel optical axes.

5. The laser device (100) of any one of claims 2 to 4, wherein the laser spectroscope (200) comprises a laser induced fluorescence spectroscope, a coherent anti-Stokes Raman scattering spectroscope, a laser photo-acoustic spectroscope, a laser induced breakdown spectroscope, a resonance-enhanced multi-photon ionization spectroscope or an elastic scattering spectroscope.

6. The laser device (100) of any one of the preceding claims, wherein the plume analyzing arrangement (250) comprises a mass spectrometer (300) or an aspirating ion-mobility spectrometer.

7. The laser device (100) of claim 6, wherein the plume analyzing arrangement (250) comprises a debris gathering unit (350) arranged to collect debris of the plume (110) generated by the ablating laser beam (402; 402i) when ablating the target tissue (120) wherein the debris gathering unit (350) is connected to the mass spectrometer (300) or the aspirating ion-mobility spectrometer.

8. The laser device (100) of claim 7, wherein the debris gathering unit (350) of the plume analyzing arrangement (250) comprises
an aspirating mouthpiece (351), and/or
a pump unit adapted to forward the debris to the mass spectrometer (300) or the aspirating ion-mobility spectrometer, and/or
an electrical field generator to collect the debris of the plume (110).

9. The laser device (100) of any one of the preceding claims, wherein the plume analyzing arrangement (250) comprises a processing unit (500) adapted to evaluate measurement data of the at least one substance in the debris of the plume (110) generated by the ablating laser beam (402) ablating the target tissue (120).

10. The laser device (100) according to any one of the preceding claims, comprising a camera (540) adapted to capture an image of the target tissue (120).

11. The laser device (100) according to any one of the preceding claims, wherein the plume analyzing arrangement is adapted to three-dimensionally localize the origin of the plume (110).

12. The laser device (100) according to claims 10 and 11, wherein the plume analyzing arrangement (250) is adapted to augment the image captured by the camera (540) with information derived from the substance in the debris of the plume (110).

13. The laser device (100) according to any one of claims 10 to 12, comprising a processing unit (500) adapted to identify a movement of the target tissue (120) relative to the laser source (401) on the captured image and to correct a position of the laser source (401) in accordance with the identified movement of the target tissue (120).

14. The laser device (100) according to any one of the preceding claims, wherein the ablation laser source (401) is three-dimensionally movable, particularly, relative to the target tissue (120).

## Patentansprüche

1. Laservorrichtung (100) mit einer Abtragungslaserquelle (401), die angepasst ist, um einen Abtragungslaserstrahl (402; 402i) zum Abtragen eines Zielgewebes (120) bereitzustellen, **gekennzeichnet durch** eine Wolkenanalyseanordnung (250), die angepasst ist, um mindestens eine Substanz, die ein Biomarker des abgetragenen Zielgewebes (120) ist, in Rückständen einer Wolke (110) zu identifizieren und/oder zu quantifizieren, die durch den Abtragungslaserstrahl (402; 402i) beim Abtragen des Zielgewebes (120) erzeugt wird.

2. Laservorrichtung (100) nach Anspruch 1, wobei die Wolkenanalyseanordnung (250) ein Laserspektroskop (200) umfasst.

3. Laservorrichtung (100) nach Anspruch 2, umfassend eine Strahlmischstruktur, wobei die Wolkenanalyseanordnung (250) eine Analyselaserquelle (403) aufweist, die angepasst ist, um einen Analyselaserstrahl (404; 404i) bereitzustellen, und die Strahlmischstruktur (470; 470i) angepasst ist, um den Abtragungslaserstrahl (402; 402i) der Abtragungslaserquelle (401) und/oder den Analyselaserstrahl (404; 404i) der Analyselaserquelle (403) derart umzulenken, dass eine optische Achse des Abtragungslaserstrahls (402; 402i) parallel zu einer optischen Achse des Analyselaserstrahls (404; 404i) ist.

4. Laservorrichtung (100) nach Anspruch 3, umfassend einen bewegbaren Scannerspiegel, der hinter der Strahlmischstruktur (470; 470i) positioniert ist, wobei der Scannerspiegel angeordnet ist, um den Analyselaserstrahl (404; 404i) und den Abtragungslaserstrahl (402; 402i) zu lenken, wenn sie parallele optische Achsen aufweisen.

5. Laservorrichtung (100) nach einem der Ansprüche 2 bis 4, wobei das Laserspektroskop (200) ein laserinduziertes Fluoreszenzspektroskop, ein kohärentes Anti-Stokes-Raman-Streuspektroskop, ein laserphotoakustisches Spektroskop, ein laserinduziertes Durchbruchspektroskop, ein resonanzverstärktes Mehrphotonenionisationsspektroskop oder ein elastisches Streuspektroskop umfasst.

6. Laservorrichtung (100) nach einem der vorhergehenden Ansprüche, wobei die Wolkenanalyseanordnung (250) ein Massenspektrometer (300) oder ein ansaugendes lonenmobilitätsspektrometer umfasst.

7. Laservorrichtung (100) nach Anspruch 6, wobei die Wolkenanalyseanordnung (250) eine Rückständesammeleinheit (350) umfasst, die angeordnet ist, um Rückstände der Wolke (110) zu sammeln, die durch den Abtragungslaserstrahl (402; 402i) beim Abtragen des Zielgewebes (120) erzeugt werden, wobei die Rückständesammeleinheit (350) mit dem Massenspektrometer (300) oder dem ansaugenden lonenmobilitätsspektrometer verbunden ist.

8. Laservorrichtung (100) nach Anspruch 7, wobei die Rückständesammeleinheit (350) der Wolkenanalyseanordnung (250) umfasst:
ein ansaugendes Mundstück (351) und/oder
eine Pumpeinheit, die angepasst ist, um die Rückstände an das Massenspektrometer (300) oder das ansaugende lonenmobilitätsspektrometer weiterzuleiten, und/oder
einen elektrischen Feldgenerator zum Sammeln der Rückstände der Wolke (110) zu sammeln.

9. Laservorrichtung (100) nach einem der vorhergehenden Ansprüche, wobei die Wolkenanalyseanordnung (250) eine Verarbeitungseinheit (500) umfasst, die angepasst ist, um Messdaten der mindestens einen Substanz in den Rückständen der Wolke (110) auszuwerten, die durch den Abtragungslaserstrahl (402) beim Abtragen des Zielgewebes (120) erzeugt wird.

10. Laservorrichtung (100) nach einem der vorhergehenden Ansprüche, umfassend eine Kamera (540), die angepasst ist, um ein Bild des Zielgewebes (120) aufzunehmen.

11. Laservorrichtung (100) nach einem der vorhergehenden Ansprüche, wobei die Wolkenanalyseanordnung angepasst ist, um den Ursprung der Wolke (110) dreidimensional zu lokalisieren.

12. Laservorrichtung (100) nach Anspruch 10 und 11, wobei die Wolkenanalyseanordnung (250) angepasst ist, um das Bild, das durch die Kamera (540) aufgenommen wird, mit Informationen zu ergänzen, die von der Substanz in den Rückständen der Wolke (110) stammen.

13. Laservorrichtung (100) nach einem der Ansprüche 10 bis 12, umfassend eine Verarbeitungseinheit (500), die angepasst ist, um auf dem aufgenommenen Bild eine Bewegung des Zielgewebes (120) relativ zu der Laserquelle (401) zu identifizieren und um eine Position der Laserquelle (401) gemäß der identifizierten Bewegung des Zielgewebes (120) zu korrigieren.

14. Laservorrichtung (100) nach einem der vorhergehenden Ansprüche, wobei die Abtragungslaserquelle (401) dreidimensional bewegbar ist, insbesondere relativ zum Zielgewebe (120).

## Revendications

1. Dispositif laser (100) doté d'une source laser d'ablation (401) adaptée pour fournir un faisceau laser d'ablation (402 ; 402i) pour l'ablation d'un tissu cible (120) **caractérisé en ce qu'**il comprend un agencement d'analyse de panache (250) adapté pour identifier et/ou quantifier au moins une substance étant un biomarqueur du tissu cible ablaté (120) dans les débris d'un panache (110) généré par le faisceau laser d'ablation (402 ; 402i) ablatant le tissu cible (120).

2. Dispositif laser (100) selon la revendication 1, dans lequel l'agencement d'analyse de panache (250) comprend un spectroscope laser (200).

3. Dispositif laser (100) selon la revendication 2, comprenant une structure de mélange de faisceaux, dans lequel l'agencement d'analyse de panache (250) présente une source laser d'analyse (403) adaptée pour fournir un faisceau laser d'analyse (404 ; 404i), et la structure de mélange de faisceaux (470 ; 470i) est adaptée pour rediriger le faisceau laser d'ablation (402 ; 402i) de la source laser d'ablation (401) et/ou le faisceau laser d'analyse (404 ; 404i) de la source laser d'analyse (403) de sorte qu'un axe optique du faisceau laser d'ablation (402 ; 402i) soit parallèle à un axe optique du faisceau laser d'analyse (404 ; 404i).

4. Dispositif laser (100) selon la revendication 3, comprenant un miroir de balayage mobile positionné après la structure de mélange de faisceaux (470 ; 470i), dans lequel le miroir de balayage est agencé pour diriger le faisceau laser d'analyse (404 ; 404i) et le faisceau laser d'ablation (402 ; 402i) lorsque leurs axes optiques sont parallèles.

5. Dispositif laser (100) selon l'une quelconque des revendications 2 à 4, dans lequel le spectroscope laser (200) comprend un spectroscope à fluorescence induite par laser, un spectroscope à diffusion Raman anti-Stokes cohérent, un spectroscope photo-acoustique laser, un spectroscope de plasma induite par laser, un spectroscope d'ionisation multi-photons à résonance améliorée ou un spectroscope à diffusion élastique.

6. Dispositif laser (100) selon l'une quelconque des revendications précédentes, dans lequel l'agencement d'analyse de panache (250) comprend un spectromètre de masse (300) ou un spectromètre à mobilité ionique par aspiration.

7. Dispositif laser (100) selon la revendication 6, dans lequel l'agencement d'analyse de panache (250) comprend une unité de collecte de débris (350) agencée pour collecter les débris du panache (110) généré par le faisceau laser d'ablation (402 ; 402i) lors de l'ablation du tissu cible (120), l'unité de collecte de débris (350) étant connectée au spectromètre de masse (300) ou au spectromètre à mobilité ionique par aspiration.

8. Dispositif laser (100) selon la revendication 7, dans lequel l'unité de collecte de débris (350) de l'agencement d'analyse de panache (250) comprend un embout buccal d'aspiration (351) et/ou une unité de pompe adaptée pour acheminer les débris vers le spectromètre de masse (300) ou le spectromètre à mobilité ionique par aspiration, et/ou un générateur de champ électrique pour collecter les débris du panache (110).

9. Dispositif laser (100) selon l'une quelconque des revendications précédentes, dans lequel l'agencement d'analyse de panache (250) comprend une unité de traitement (500) adaptée pour évaluer les données de mesure de l'au moins une substance dans les débris du panache (110) généré par le faisceau laser d'ablation (402) ablatant le tissu cible (120).

10. Dispositif laser (100) selon l'une quelconque des revendications précédentes, comprenant une caméra (540) adaptée pour capturer une image du tissu cible (120).

11. Dispositif laser (100) selon l'une quelconque des revendications précédentes, dans lequel l'agencement d'analyse de panache est adapté pour localiser en trois dimensions l'origine du panache (110).

12. Dispositif laser (100) selon les revendications 10 et 11, dans lequel l'agencement d'analyse de panache (250) est adapté pour augmenter l'image capturée par la caméra (540) avec des informations dérivées de la substance dans les débris du panache (110).

13. Dispositif laser (100) selon l'une quelconque des revendications 10 à 12, comprenant une unité de traitement (500) adaptée pour identifier un mouvement du tissu cible (120) par rapport à la source laser (401) sur l'image capturée et pour corriger une position de la source laser (401) conformément au mouvement identifié du tissu cible (120).

14. Dispositif laser (100) selon l'une quelconque des revendications précédentes, dans lequel la source laser d'ablation (401) est mobile en trois dimensions, en particulier, par rapport au tissu cible (120).
